Europäisches Patentamt

European Patent Office

Office européen des brevets

(11) Publication number: **0 503 682 A1**

# EUROPEAN PATENT APPLICATION

(21) Application number: 92107525.5

(22) Date of filing: 04.05.89

(51) Int. Cl.⁵: **A61K 35/16**, A61M 1/36

This application was filed on 04 - 05 - 1992 as a divisional application to the application mentioned under INID code 60.

(30) Priority: **06.05.88 US 191039**
**13.09.88 US 243786**
**20.10.88 US 260382**

(43) Date of publication of application:
**16.09.92 Bulletin 92/38**

(60) Publication number of the earlier application in accordance with Art.76 EPC: **0 345 943**

(84) Designated Contracting States:
**AT BE CH DE ES FR GB GR IT LI LU NL SE**

(71) Applicant: **APPLIED IMMUNESCIENCES, INC.**
**200 Constitution Drive**
**Menlo Park California 94025-1109(US)**

(72) Inventor: **Okarma, Thomas B.**
**1651 Portola**
**Palo Alto, California 94306(US)**
Inventor: **Chang, Chin-Hai**
**977 Glennan Drive**
**Redwood City, California 94061(US)**
Inventor: **Clark Brian R.**
**1332B Mills Street**
**Menlo Park, California 94025(US)**
Inventor: **Lerch, Bernard L.**
**1110 Runnymead Drive**
**Los Altos, California 94022(US)**

(74) Representative: **Harrison, David Christopher et al**
**MEWBURN ELLIS 2 Cursitor Street**
**London EC4A 1BO(GB)**

(54) Device for plasma modification composition and remodeling.

(57) A method of reducing the elevated levels of anaphylatoxins found in blood derived fluid by contacting the fluid with silicic acid is provided.

EP 0 503 682 A1

The subject invention is concerned with plasma processing devices involving separation of soluble plasma components and remodeling of soluble plasma components.

Blood and lymph are the fluid highways of the body. These fluids provide for the transport of nutrients, metabolites, growth factors, hormones, and the like, from one site in the body to another, allowing for production of various compounds by cells and tissues in one part of the body, for the regulation of cells and tissues in another part of the body. In addition, these fluids allow for removal of waste materials, so as to prevent the accumulation of compounds which could interfere with the ability of cells and organs to function. Of equal importance is the fact that these fluids also allow for transport of cells of the hematopoietic system throughout the body to fulfill their variegated function, while also bringing a wide variety of materials to cells of the hematopoietic system for processing or for inducing a cellular response, as in the case of antigens, pathogens, or the like.

In many situations, the interaction between substances in the blood and hematopoietic cells can result in products which may provide fruitful information about the diseased state of the individual, provide compositions of interest in relation to the host or other individuals, or cause adverse affects to the host. There is, therefore, a substantial interest in accessing these fluids and isolating, identifying or remodeling various components in the blood stream.

Blood, however, is an extraordinarily complex mixture, which may respond to an alien environment in a wide variety of ways. Commonly, blood clots result in the stoppage of flow. Where plasma is used, contact with foreign materials can activate various blood components resulting in substantial changes in the blood composition. While this may not be a problem in many instances where the blood or plasma is not being reused, when the blood is to be returned to the host, such changes many detrimentally affect the host and therefore preclude the reuse of the blood.

In many instances, it is desirable to restore a person's blood, such as in plasmapheresis, because of the uncertainties concerning the safety of the blood supply, due to hepatitis, HIV, HTLV-I, or the like, or the availability of the correct blood type.

It is therefore of interest to develop procedures and equipment which allow for selective treatment of blood or plasma, by modification of the nature and/or amount of components of the blood and saving the blood for restoration to the host from which the blood was withdrawn. Necessary for this purpose is identification of materials, components, and conditions which allow for such selective treatment without significant adverse effects on the blood.

Relevant Literature

Descriptions of blood component removal systems may be found in U.S. Patent Nos. 4,086,924; 4,103,685, 4,223,672; 4,362,155; 4,428,744; 4,464,165; 4,540,401; 4,614,513; 4,627,915 and Re 31,688 and EPA 0 082 345. References associated with complement activation include Breillatt and Dorson, ASAIO J. - (1984) 7:57-63 and McLeod et al., Artif. Organs (1983) 7.443-449.

U.S. 4,551,435 (Liberti et al) discloses a method for removing immunospecifically recognized substances by finding out the amount of substance present, forming immune complexes with the substance, said immune complexes then being bound to an absorbent.

SUMMARY OF THE INVENTION

Devices for the modification of blood or plasma involving the removal or remodeling of blood components are described in the parent application, EP 345943. The present application provides a method of reducing elevated levels of anaphylatoxins in blood derived fluid or by contacting the fluid with silicic acid. The blood derived fluid may be serum or plasma.

BRIEF DESCRIPTION OF THE DRAWINGS

Fig. 1 is a diagrammatic view of a device according to this invention;
Fig. 2 is an exploded perspective view of a box device and its contents according to the invention;
Fig. 3 is a cross-sectional elevation of a tubular device according to this invention; and
Fig. 4 is a perspective view of the parts of the device of Fig. 3.

It is found that when using the device of EP 345943 that the formation of specific binding protein complexes results in a great enhancement in complement activation to anaphylatoxins. Since the anaphylatoxins can be detrimental if the plasma is restored to the host, the subject device will normally be employed with means for reducing a dangerous level of anaphylatoxins to a safe level. It is found that this can be achieved by passing the fluid from the subject device to a chamber containing silicic acid particles, where the silicic acid is found to substantially remove dangerous levels of anaphylatoxin, without a significantly deleterious effect on other components of the blood or the plasma characteristics. The anaphylatoxin removing device is described in copending U.S. Patent No. 4,963,265, filed May 6, 1988. The disclosure of this application is specifically incorporated herein by reference.

Briefly, the silicic acid particles will generally be of a size in the range of about 50 to 500 $\mu$m, and of neutral and acidic pH in the range of about 3 to 7. The pore size will generally be in the range of about 5 to 35 nm (50 to 350 Å) with a surface area of at least about 200 m$^2$/g. The fluid stream, particularly plasma, from the subject device will generally be directed directly into the anaphylatoxin removing device.

The ratio of silicic acid to fluid generally will be in the range of about 10 to 100 g/L of fluid, more usually from about 15 to 50 g/L of fluid. The temperature will generally range from about 20 to 40°C, preferably from about 25 to 37°C. Ambient temperatures will usually be convenient.

After the plasma has circulated through the device of EP 345943 and, as appropriate, been further treated to remove any anaphylatoxins, it may then be restored to the host in accordance with conventional techniques. Thus, the plasma may be removed and returned in a continuous manner or discontinuous manner, as appropriate.

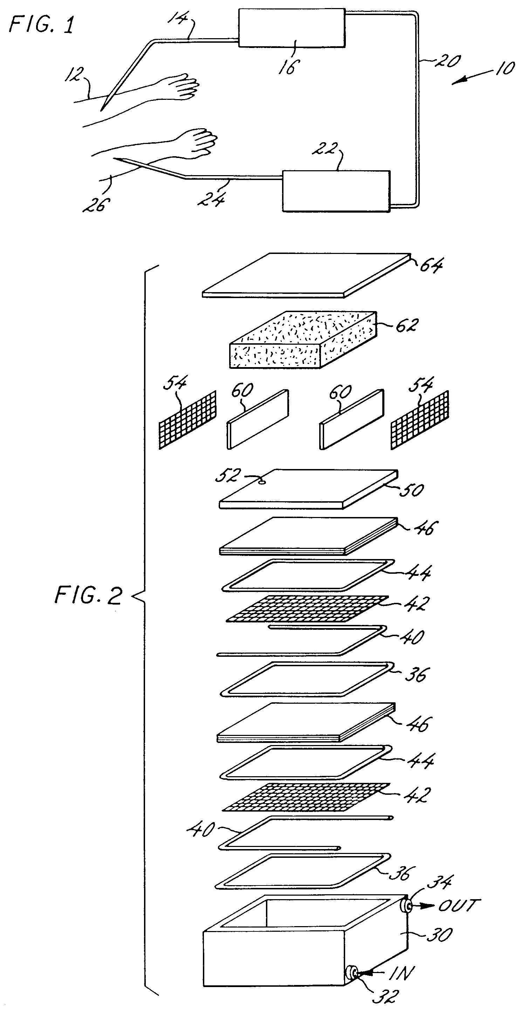

To further understand the subject invention, the drawings will now be considered. In Fig. 1, a schematic of the subject device is provided. The device 10 receives blood from one arm 12 of a patient through conduit 14. Conduit 14 introduces the blood into the first chamber 16, where one or more components may be exchanged, removed, or otherwise modified. The blood exits into conduit 20 and is directed by conduit 20 to anaphylatoxin removal chamber 22. The modified blood free of an undesirable level of anaphylatoxins is then directed through conduit 24 to the other arm 26 of the patient. In this manner, the blood has been modified in accordance with the needs of the patient and is returned to the patient free of elevated levels of anaphylatoxins to avoid potential shock.

In Fig. 2 is indicated an exploded view of a device in the shape of a box having first and second compartments where the first compartment has a plurality of membranes overlying one another and the second compartment has the silicic acid. The membrane compartment provides for an alternating direction of flow of the blood derived stream through the compartment. The device has a housing 30 with inlet 32 and outlet 34. Contained in the membrane compartment is O-ring 36, U-ring 40, and screen 42. The U-ring controls the direction of flow of the stream. On top of the screen 42 is a second O-ring 44 which separates the O-ring from membrane pack 46. The membrane employed may be Nalgene affinity chromatography unit bound with protein A, U12A or U38A (cat. nos. 751-2012 and 751-5038). The membrane pack will have a plurality of membranes lying one atop the other to which will be bound the specific binding pair members. Conveniently, each pack may contain from about 5 to 25, usually 5 to 20, membranes. The blood derived stream will pass up through the membrane pack 46 contacting the specific binding pair members and rising up through the pores to repetitively contact each succeeding membrane. Once the blood derived stream has passed through the membrane pack, the assemblage of O-ring 36, U-ring 40, positioned in the opposite direction of the previous Uring 40, O-ring 36, screen 42, second O-ring 44 and membrane pack 46 may be repeated one or more times depending upon the size of the unit, the amount of material to be extracted, the binding capacity of the membrane packs and the like. The particular component which is the last component is not critical to this invention.

Various biocompatible materials may be employed for the various components. Conveniently, the O-ring and U-spacers may be high density polypropylene, the screens polypropylene and the housing polycarbonate.

Surmounting the components of the membrane compartment will be an inner lid 50 having port 52. The port 52 will be of approximately the same dimensions as the inlet and outlet ports 32 and 34 respectively of the housing 30. A polyethylene filter, not shown, conveniently of a pore size of 35-60 $\mu$m is applied across the port to prevent access of silicic acid particles into the membrane pack compartment. Barriers 54 and 60 are employed to maintain the silicic acid within a predetermined area in the silicic acid compartment. The silicic acid particles are indicated as a box 62. The silicic acid particles may be of a size in the range of from about 50 to 300 $\mu$m. A cover 64 is then used to close the housing 30 completing the device.

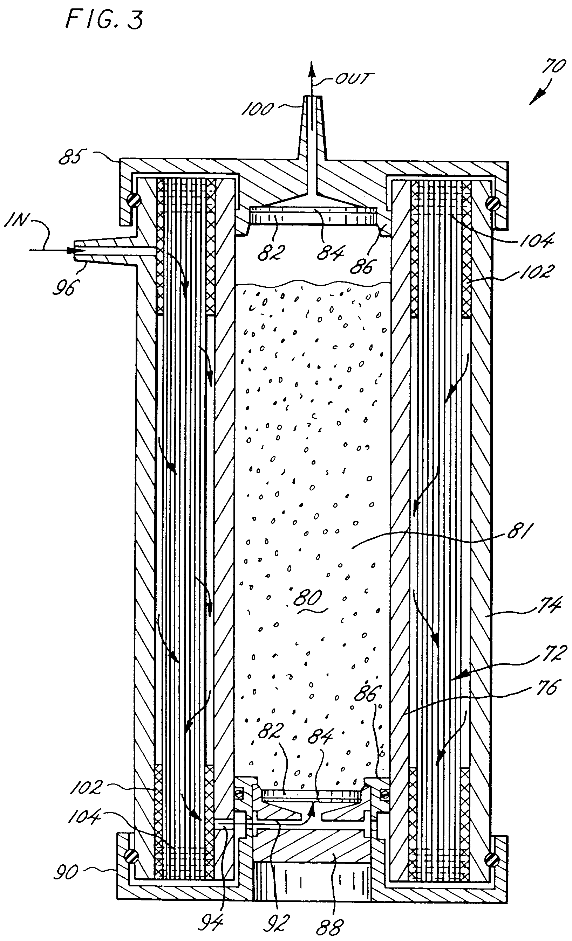

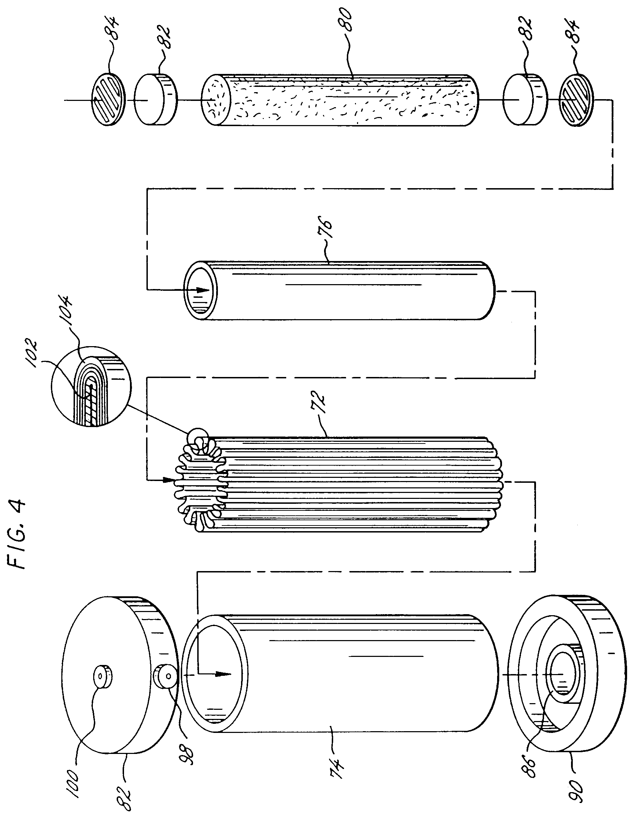

A third device is depicted in Figs. 3 and 4. The device 70 is cylindrical, having cylindrical membrane 72 fitted into cylinder 74 which serves as the membrane compartment. An inner tube or sleeve 76 serves for mounting the membrane 72 and to define the silicic acid compartment 80. Silicic acid particles 81 as described previously are then packed into silicic acid compartment 80. First and second screens 82 and 84 respectively are mounted at the bottom and top of compartment 80 to ensure that silicic acid particles do not escape.

The device may be assembled by employing top cap 85 and bottom cap 90 and mounting inner tube 76 on projection 86 which holds the inner tube 76 in place. Included within inner tube 76 is mounting 90 which includes conduit 92, which is in alignment with orifice 94 in innertube 76. Mounting 90 receives and holds first and second screens 82 and 84 in position to prevent the silicic acid particles 81 from entering the membrane compartment 74. The top cap 85 has plasma inlet 96 and plasma outlet 100.

After mounting the inner tube 76 on projection 86, membrane 72 is then fitted onto inner tube 76, followed by mounting of membrane compartment tube 74 which encloses membrane 72. Assemblage of the device is completed by adding the upper silicic acid screens 82 and 84 over the silicic acid, followed by enclosing the device with top cap 85 which includes plasma inlet orifice 96 and plasma outlet 100.

The top of the membrane may be coated with netting 102 which is held in place with a hot melt 104 so as to provide structural stability to the membrane 72.

In using the device, the plasma will enter inlet 96 and flow downwardly through membrane 72. The flow of plasma will be circular around the device, filling the membrane with the plasma. The plasma will reach the bottom of the device and pass through orifice 94 into conduit 92. From conduit 92, the plasma will pass through first and second screens 82 and 84 into the silicic acid particles 81, where anaphylatoxins will be removed. After passing upwardly through the silicic acid particles 81, the plasma will pass through upper screens 82 and 84 through outlet 100.

The membrane may then be easily removed for regeneration or other use by removing the top cap 85 and the membrane compartment 74 and retrieving the membrane 72 by removal from the sleeve or inner tube 76.

Other equipment may be employed with the device, including additional extraction systems. Usually, a pump or an hydraulic device will be employed to move the blood from the patient or other source through the various compartments and conduits. Various alarm and control systems may be employed for detecting rate of flow, flow blockages, air bubbles, clots, or the like. Other components may be additional filters, absorbents, chemical treatments, radiation treatments, and the like. Various electronic equipment may be associated with the device to provide for the automation of various fluid flows, the eluent where the bound material is removed, and the like.

The following examples are for illustration and not by way of limitation.

The device as depicted in Figure 1 is prepared as follows. A polycarbonate case encloses ten packs of cellulosic membranes, each pack contains ten rectangular sheets of membrane. Each pack is separated from the next by a polypropylene O-ring and a flowdirecting U-ring, as well as the polypropylene separation screens. The ten packs are compressed beneath a polycarbonate lid having an access port equal in diameter to the inlet and outlet ports of the polycarbonate case. A 35-60 $\mu$m high-density polyethylene filter is applied across the access port to prevent entry of silicic acid particles into the compartment containing the membrane packs. The same filter is placed across the outlet of the silicic acid compartment to prevent the silicic acid from being entrained with the plasma.

The silicic acid has a particle size of 75-250 $\mu$m. The polycarbonate lid is then applied.

The receptor is then covalently immobilized to the membrane as described below. By the process of immobilization described below, approximately 400 mg of recombinant protein A (rPA) is covalently attached to the membrane prior to the addition of the silicic acid to the silicic acid compartment. In this manner the subject device contains ten square feet of cellulosic membrane to which is bound approximately 400 mg rPA.

The preparation and characterization of the membrane is described in the parent application EP 345943 as is the immobilization of the protein on the membrane.

In order to determine whether the covalently bound protein is substantially free of leaching when perfused with plasma or another aqueous medium, the following experiments were carried out. Four ethylene oxide-sterilized prototype devices having polyethylene silicic acid filters, ten square feet of membrane, 400 mg of rPA and 90 g of silicic acid were subjected to the following flush/perfusion protocol. Two 500 ml volumes of 0.9% saline at room temperature were pumped through the device at 200 ml/min and collected separately. A sample was taken of each. Subsequently, two additional 500 ml volumes of 0.9% saline at 37°C were sequentially pumped through each device at 200 ml/min and collected separately. A sample was taken of each.

An additional 500 ml of 0.9% saline at 37°C was recirculated continuously through the device at 50 ml/min for 4 h. At the end of this procedure, the saline perfusate was collected separately and a sample taken for assay. All samples were assayed by an rPA specific ELISA assay and by less specific methods (BCA, biocinchoninic acid, and optical density at 210 and 280 nm). The results are indicated in Table 1.

The BCA active material was established to be a contaminant of the assembly used in the flush/ perfusion protocol by employing a sham procedure using a tubing and pump assembly identical to the flush/perfusion study, except that the device was not included in the loop. The data showed that negligible (<0.001%) amounts of rPA were detected in eluates from the device during perfusion.

To determine the specificity of binding of the subject device, the following experiments were carried out. Two devices were studied, one with silicic acid and one without silicic acid, where a polyethylene filter was used as described previously. All the devices were sterilized with ethylene oxide. The protocol was as follows.

After priming the device with 1 L of a phosphate buffer saline flush, 1.5 L normal human plasma was pumped through the device at 50 ml/min in a single pass. Pre- and post-perfusion plasma samples were retained. Earlier data had shown that protein A is saturated by approximately 1-1.5 L of normal plasma. Two liter phosphate buffered saline flushes were then followed by two 0.5 M acetic acid 2 L flushes and samples of the acetic acid washes saved. Both the acetic acid wash samples and the pre/post-plasma were

## Table 1

### LEACHING OF PROTEIN A BY FLUSH/PERFUSION

### Four devices having internal polyethylene silicic acid filters.

| Device Item Number | Sample | ELISA (ng/ml) | BCA (mg) | 210 nm (OD units) | 280 nm (OD units) |
|---|---|---|---|---|---|
| 1987 | Flush 1 | <10 | 3.4 | .274 | .031 |
|  | Flush 2 | <80 | 0 | .131 | .014 |
|  | Flush 3 | 85 | 0 | .090 | .012 |
|  | Flush 4 | 10 | 0 | .056 | .009 |
|  | Perfusion | 96 | 1.55 | .242 | .023 |
| 1990 | Flush 1 | 10 | 0 | .442 | .036 |
|  | Flush 2 | <80 | 0 | .141 | .020 |
|  | Flush 3 | 83 | 0 | .084 | .017 |
|  | Flush 4 | 11 | 0 | .058 | .013 |
|  | Perfusion | 109 | 0 | .144 | .023 |
| 2000 | Flush 1 | 12 | 0 | .178 | .089 |
|  | Flush 2 | <80 | 0 | .098 | .016 |
|  | Flush 3 | <80 | 0 | .060 | .009 |
|  | Flush 4 | 12 | 0 | .034 | .006 |
|  | Perfusion | 80 | 0 | .116 | .01 |
| 2004 | Flush 1 | 11 | 0 | .186 | .014 |
|  | Flush 2 | <80 | 0 | .116 | .004 |
|  | Flush 3 | 115 | 0 | .055 | .005 |
|  | Flush 4 | 13 | 0 | .039 | .001 |
|  | Perfusion | 80 | 1.35 | .107 | .007 |

analyzed by a Bradford assay kit (Bio-Rad 500-001). The difference between pre- and post- samples minus that contained in residual plasma represents total IgG binding, while the specifically bound IgG is the result obtained from the acetic acid washes. This is also the recoverable IgG.

Table 2 indicates the results.

Table 2

IgG BINDING BY PROTOTYPE DEVICES

$1\cdot39\,m^2$

A. IgG binding by (15 ft$^2$) device having 750 μ rPA* but no silicic acid compartments.

| Device Item Number | Specificity Binding (grams) | Total IgG Binding (grams) |
|---|---|---|
| 1767 | 0.874 | 2.64 |
| 1768 | 0.910 | 3.51 |
| 1747 | 1.015 | 1.49 |
| 1753 | 0.80 | 7.50 |
| 1734 | 1.246 | 5.05 |
| 1737 | 1.326 | 3.96 |
| 1718 | 1.540 | 3.00 |
| mean | 1.10 | 3.88 |

B. IgG binding by assembled device having polyethylene filters* (10 ft$^2$, 400 mg rPA, 90 gm silicic acid).

* $0\cdot43\,m^2$

| Device Item Number | Specificity Binding (grams) | Total IgG Binding (grams) |
|---|---|---|
| 1987 | 1.037 | 2.685 |
| 1990 | 0.959 | 2.550 |
| 2000 | 0.805 | 3.735 |
| 2004 | 0.912 | 2.390 |
| mean | 0.928 | 2.84 |

* rPA = recombinant Protein A.

The above results demonstrate that the subject device, with or without the silicic acid compartment, effectively and consistently binds IgG from human plasma. Based on the above data, a mean value of approximately 3.0 g total IgG is bound from 1500 ml of processed plasma.

**Claims**

1. A method for reducing elevated levels of anaphylatoxins in a blood derived fluid consisting of serum or plasma by contacting the fluid with an anaphylatoxin reducing amount of silicic acid.

2. A method according to claim 1, wherein said silicic acid is characterized by having a pH in the range of about 4 to 7, a size in the range of about 50 to 400 $\mu$m, a pore size in the range of about 5 to 35 nm (50 to 350 A), and a surface area of at least about 200 m$^2$/g.

FIG. 1

FIG. 2

OUT

IN

FIG. 3

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 267 047 (W. HENNE ET AL.)<br>* column 5, line 41 - line 44 * | 1-2 | A61K35/16<br>A61M1/36 |
| Y | BIOCHEMICAL PHARMACOLOGY<br>vol. 29, no. 3, 1980, LONDON UK<br>pages 319 - 323;<br>C.J. WHELAN: 'The partial purification of slow reacting substance of anaphylaxis from rat peritoneal anaphylactic fluid and its separation from an arachidonic acid releasing substance.'<br>* page 320, column 1, line 17 - line 26 *<br>* page 321, column 1, line 8 - column 2, line 2 * | 1-2 | |
| T | BLOOD PURIFICATION<br>vol. 6, no. 6, 1 June 1988, BASEL CH<br>pages 325 - 335;<br>S. JERSTAD ET AL.: 'Generation and removal of anaphylatoxins during hemofiltration with five different membranes.'<br>* the whole document * | 1-2 | |

| | |
|---|---|
| | TECHNICAL FIELDS SEARCHED (Int. Cl.4) |
| | A61K<br>A61M |

The present search report has been drawn up for all claims

| Place of search | Date of completion of the search | Examiner |
|---|---|---|
| THE HAGUE | 13 JULY 1992 | VAN BOHEMEN C.G. |